# EUROPÄISCHE PATENTSCHRIFT

(11) **EP 2 170 203 B1**
(45) Veröffentlichungstag und Bekanntmachung des Hinweises auf die Patenterteilung: **14.08.2013**
(21) Anmeldenummer: 08773460.4
(22) Anmeldetag: 16.06.2008
(51) Int. Cl.: A61B 18/24

(54) **UROLOGISCHE DIODENLASERSYTEME MIT GLASFASER-APPLIKATIONSSYSTEM**
UROLOGICAL DIODE LASER SYSTEMS HAVING A GLASS-FIBER APPLICATION SYSTEM
SYSTÈMES DE LASER À DIODE UROLOGIQUES COMPRENANT UN SYSTÈME D'APPLICATION À FIBRE DE VERRE

(30) Priorität: 15.06.2007 DE 202007008378 U
(43) Veröffentlichungstag der Anmeldung: 07.04.2010
(73) Patentinhaber: Biolitec Pharma Marketing Ltd., F.T. Labuan (MY)
(72) Erfinder: NEUBERGER, Wolfgang, 53121 Bonn (DE); SPANIOL, Stefan, 53121 Bonn (DE); SANDROCK, Thomas, 53121 Bonn (DE); GROENHOFF, Endrik, 53121 Bonn (DE)
(74) Vertreter: Lang, Johannes
(86) Internationale Anmeldenummer: PCT/EP2008/004826
(87) Internationale Veröffentlichungsnummer: WO 2008/151840

(56) Entgegenhaltungen:
- WO-A2-2007/058891
- WO-A2-2007/064783
- DE-A1- 19 703 208
- US-A- 5 322 507
- US-A1- 2004 236 318
- ANVARI B ET AL: "TREATMENT OF BENIGN PROSTATIC HYPERPLASIA USING A HIGH POWER DIODE LASER" BIOMECHANICS, REHABILITATION, ELECTRICAL PHENOMENA, BIOMATERIALS. SAN DIEGO, OCT. 28 - 31, 1993; [PROCEEDINGS OF THE ANNUAL INTERNATIONAL CONFERENCE OF THE ENGINEERING IN MEDICINE AND BIOLOGY SOCIETY], NEW YORK, IEEE, US, Bd. 15 PART 03, 28. Oktober 1993 (1993-10-28) , XP000452989

## Beschreibung

Die vorliegende Erfindung bezieht sich auf das Gebiet der medizinischen Behandlung mit Laserenergie. Sie bezieht sich des Weiteren auf die Erzeugung von geeigneter Laserstrahlung, deren Übertragung über einen Lichtwellenleiter sowie der therapeutischen Nutzung der durch den Lichtwellenleiter übertragenen Laserstrahlung zur selektiven Bearbeitung urologischen Gewebes. Im Speziellen ist die vorliegende Erfindung zur medizinischen Behandlung der gutartigen Prostatavergößerung (benignen Prostatahyperplasie, BPH) mittels selektiver endoskopischer Vaporisation geeignet.

Benigne Prostatahyperplasie (BPH) oder "(gutartige) Prostatavergrößerung" bezeichnet das nicht krebsartige (benigne) Wachstum der Prostatadrüse. Während BPH das am Meisten verbreitete Prostataproblem bei über 50-jährigen Männern darstellt, beginnt das Wachstum der Prostata mit mikroskopischen Knötchen (Noduli) im Alter von etwa 25 Jahren, führt jedoch selten zu Symptomen vor Erreichung des 40. Lebensjahres. Allein in den USA leiden schätzungsweise 6,3 Millionen Männer unter BPH. Die Krankheit ist jährlich verantwortlich für 6,4 Millionen Arztbesuche und über 400000 Krankenhauseinweisungen.

Die genaue Ursache für BPH ist unbekannt, wird jedoch in Zusammenhang mit altersbedingten Hormonveränderungen gesehen. Testosteron scheint eine Rolle bei der BPH zu spielen, da es während der gesamten Lebensdauer eines Mannes kontinuierlich produziert wird und eine Vorstufe des Dihydrotestosterons (DHT) ist, welches das schnelle Wachstum der Prostatadrüse während der Pubertät und dem frühen Erwachsenenalter verursacht. Im voll entwickelten Zustand besitzt die Prostatadrüse in etwa Walnussgröße und behält diese Größe bis zur Mitte des fünften Lebensjahrzehntes. Ab diesem Zeitpunkt beginnt eine zweite Wachstumsphase der Prostata, die für viele Männer häufig zu einem späteren Zeitpunkt ihres Lebens zu BPH führt.

Im Gegensatz zur allgemeinen Vergrößerung der Drüse im frühen Erwachsenenalter vollzieht sich die gutartige Prostatavergrößerung ausschließlich im Zentralbereich der Drüse, der so genannten Transitionszone bzw. dem Übergangsbereich, welcher die Harnröhre umschließt. Sobald dieser Bereich der Prostata beginnt zu wachsen, drückt die Drüse auf die Harnröhre und verursacht eine Reihe von Symptomen der unteren Harnwege (lower urinary tract symptoms - LUTS) wie zum Beispiel Schwierigkeiten und Schmerzen beim Urinieren (obstruktive Symptome und Speichersymptome). Schließlich wird die Harnblase selbst immer schwächer und verliert die Fähigkeit, sich zu leeren.

Obstruktive Symptome wie stoßweiser oder verzögerter Harnfluss können das vom Körper ausgeschiedene Urinvolumen schwerwiegend verringern. Bei ausbleibender Behandlung kann akute Harnverhaltung zu anderen schweren Komplikationen wie Blasensteinen, Infektionen des Harntraktes, Inkontinenz, sowie in seltenen Fällen zu Blasen- und Nierenschädigungen führen. Diese Komplikationen treten häufiger bei älteren Männern auf, die gleichzeitig Antiarrhythmika oder antihypertensive (antidiuretische/harnhemmende) Medikamente einnehmen. Zusätzlich zu den körperlichen / physischen Problemen, die mit BPH verbunden sind, leiden viele Männer unter Angstgefühlen und einer verringerten Lebensqualität.

Anfängliche Symptome der BPH werden meistens medikamentös zum Beispiel. mit Alphablockern oder Anti-Androgenen behandelt. Männer mit moderaten bis starken BPH-Beschwerden werden typscherweise operiert. Das Standardverfahren hierfür ist die transurethrale Resektion der Prostata (TURP), welches jedoch unter Umständen einige Probleme mit sich bringt:
- Blutung bzw. Nachblutung
   Die geringe Eindringtiefe verbunden mit der Entfernung großer Volumina führt zu Öffnung vieler Gefäße, die in die Operationshöhle hinein bluten. In etwa 1% der Fälle kommt es zu so starken Blutungen, dass sogar eine Bluttransfusion erforderlich wird. Teilweise kann, bedingt durch eine Nachblutung, eine operative Blutstillung mit erneuter Narkose notwendig werden. In jedem Fall muss dauerhaft gespült werden, um eine freie Sicht zu haben. Des Weiteren werden die Patienten postoperativ für 5 bis 7 Tage im Krankenhaus beaufsichtigt, um bei eventuell auftretenden Nachblutungen gerüstet zu sein. Selbst Husten kann eine solche Nachblutung hervorrufen.
- "TURP-Syndrom"
   Im Resektoskop befindet sich eine Drahtschlinge über die ein elektrischer Strom fließt. Damit wird erkranktes Gewebe in der Harnblase oder Prostata schichtweise abgetragen. Auftretende Blutungen werden elektrisch verödet (Kauterisierung). Das physikalische Prinzip entspricht dem in der HF-Chirurgie angewandten. Während dieser Operation wird permanent eine Spülflüssigkeit über das Resektoskop eingebracht. Sie dient zum einen der Blasenfüllung und zum anderen der Ausschwemmung von reseziertem Gewebe und Blut. Diese Lösung ist hypoton, das heißt, sie hat eine geringere Elektrolytkonzentration als das Blut. Die geringe Elektrolytkonzentration ist in der Notwendigkeit einer geringen Leitfähigkeit begründet. Durch das Einspülen von Wasser in geöffnete Blutgefäße kann es zu Verschiebungen im Salz- und Wasserhaushalt kommen ("Einschwemmung"). Die Folge ist eine Herz-Kreislauf-Belastung bis hin zu akuter Rechtsherzinsuffizienz. Diese können gar zum Tode führen. Symptome sind Übelkeit, Erbrechen, Verwirrtheit und Unruhe.
- Inkontinenz, das heißt, die Unfähigkeit, den Urin willentlich zu halten Die Angaben zur Häufigkeit variieren stark. Die Komplikation ist bei einem geübten Operateur selten (ca. 1%). Andererseits kann sich eine durch die BPH bedingte Inkontinenz nach der Therapie bessern.
- Impotenz
   Auch hier variieren die Angaben stark (10 - 15%). Eine mögliche Ursache ist die Schädigung der Nerven an der Kapselaußenseite durch Strom, auch seelische Ursachen werden diskutiert. Insgesamt sind auch hiervon wenige Patienten betroffen. Studien zur Impotenz bei einer BPH weisen darauf hin, dass unter verschiedenen Therapien (auch unter Zuwarten) Impotenz auftrat.
- Blasenentzündungen und Entzündungen der Nebenhoden kommen häufiger vor, sind aber mit Antibiotika gut zu behandeln.
- Entleeren der Samenflüssigkeit in die Blase (60 - 80%)
- Perforation der Prostatakapsel bzw. des Rektums

Mögliche Spätfolgen sind:
- erektile Dysfunktion (Erektionsstörung)
- retrograde Ejakulation (kein Samenerguss)
- Monate bis Jahre nach der Therapie kann es zum Auftreten von narbigen Harnröhrenengen und zu einer Verengung des Blasenhalses kommen. Auch eine erneute Adenombildung ist möglich.

Mittlerweile gibt es jedoch einige weitere, weniger invasive Verfahren: transurethrale Inzision der Prostata (TUIP), transurethrale Mikrowellen-Thermotherapie (TUMT), transurethrale Elektrovaporisation der Prostata (TUVP) transurethrale Nadelablation (TUNA) und Laserchirurgie der Prostata.

Bei der TUMT wird das Zielgewebe über eine Mikrowellensonde erwärmt. Da lediglich eine Sedierung notwendig ist, bietet sich das Verfahren für Patienten an, für die eine Narkose ein hohes Risiko darstellen würde. Dies ist jedoch nur bei kleinem Prostatavolumen möglich. Kontraindiziert ist die TUMT bei Trägern von Herzschrittmachern oder metallhaltigem Osteosynthesematerial.

Eine TUNA wird ebenfalls nur unter Lokalanästhesie durchgeführt werden, damit kann sie auch bei Hochrisikopatienten durchgeführt werden. Sie ist jedoch nur für Prostatavolumina < 60ml geeignet. Für die Effektivität des Verfahrens liegen zur Zeit keine gesicherten Daten vor. In bis zu 14% aller Fälle ist eine erneute Operation erforderlich.

Bei der Adenomenukleation (Entfernung des Prostatagewebe durch chirurgische Schnitte) können auch große Prostatavolumina von mehr als 75ml entfernt werden. Dieser Eingriff kann ebenfalls angezeigt sein bei symptomatischem Blasendivertikel (Ausstülpung der Blasenwand nach Außen) oder großem Blasenstein. Von Vorteil ist, dass der Operateur direkte Sicht auf den Blasenauslass hat. Somit lassen sich Blasenverletzungen sicher vermeiden. Adenome lassen sich komplett entfernen. Die Komplikationen eines TURP-Syndroms treten nicht auf. Wie bei allen "offenen Operationen" erfordert dieses Verfahren jedoch einen längeren stationären Krankenhausaufenthalt.

Heute werden die oben genannten urologischen Indikationen alternativ mittels Laserstrahlung therapiert. Im Vergleich zu den "klassischen" Therapieformen bieten diese Verfahren den Vorteil einer überlegenen Hämostase (Blutstillung) sowie eines minimalinvasiven Eingriffs.

Zu dieser Therapie mittels Laserstrahlen werden derzeit verschiedene Systeme mit Festkörperlasern, diodengepumpten Festkörperlasern (DPSSL, diode-pumped solid-state laser) oder frequenzverdoppelten DPSSL eingesetzt. Hierbei wird durch Blitzlampen oder Laserdioden Licht erzeugt, das wiederum zur Anregung des jeweiligen Festkörperlasers verwendet wird. Im Folgenden werden nun einige Beispiele derartiger Systeme und der darin verwendeten Festkörperlaser beschrieben.

Der sogenannte KTP-Laser (Kalium-Titanyl-Phosphat-Laser), bei dem es sich um einen frequenzverdoppelten Festkörperlaser mit 532nm Wellenlänge handelt, wirkt primär ablativ, das heißt, Zielgewebe wird vaporisiert. Typische Laserparameter sind Pulsdauern von einigen Mikro- bis einigen Millisekunden sowie - Anwendungen im Dauerbetrieb ("continuous wave", cw) bei mittleren Leistungen von ca. 80W. Von Vorteil bei dieser Therapieform ist, dass der Patient nur kurzzeitig, das heißt, für 1 bis 2 Tage einen Blasenkatheter benötigt. Nachteilig sind die hohen Kosten für Laser und glasfaserbasiertes Applikationssystem. Des Weiteren besteht die Gefahr der Perforation der Blasenwand, die unter Umständen, bei Bruch der Glasfaser letale Folgen haben kann.

Sekundär ablativ (koagulierend) wirken Nd:YAG-Laser (neodymdotierte Yttrium-Aluminium-Granat-Laser) mit einer Emissionswellenlänge von 1064nm, die am Gewebe Nekrosen verursachen, die innerhalb von vier Wochen abschilfern. Während dieser Zeit muss der Patient einen Blasenkatheter dulden, das heißt, der Heilungsprozess ist langwierig und der Patient erfährt keine unmittelbare Linderung seiner Beschwerden. Typische Behandlungsparameter sind Pulsdauern von einigen Mikro- bis einigen Millisekunden sowie cw-Anwendungen bei mittleren Leistungen von ca. 80W.

Eine interstitielle Laserkoagulation, das heißt, eine Koagulation im Gewebe, führt zur Verkleinerung des Adenomvolumens über atrophischen und narbigen Umbau. Da kein Gewebe entfernt wird, tritt eine Besserung nicht sofort ein. Der Eingriff ist zwar minimalinvasiv, verursacht aber höhere Kosten.

Allen diesen Systemen ist der Nachteil gemein, dass sie prinzipbedingt neben dem Festkörperlaser eine Lichtquelle benötigen, was zu einer relativ ineffizienten Umsetzung von elektrischer Speiseleistung in Laserleistung ("wall plug efficiency") führt. Die beschriebenen Laser sind zumeist mit einem Drehstromanschluss versehen und benötigen häufig eine Wasserkühlung. Selbst aktuelle Systeme für den Betrieb an nur einer Phase benötigen eine Absicherung bis 32A. Dies erfordert, dass zum Betrieb dieser Systeme spezielle elektrische Stromversorgungsinstallationen entsprechender Leistung vorgesehen werden müssen, was einen mobilen Einsatz dieser Systeme verhindert. Auch das Gewicht solcher Systeme von ca. 140kg und mehr, sowie die Anforderungen an das Kühlmedium stehen einem mobilen Einsatz entgegen.

Zudem erfordert der komplexe Aufbau solcher Systeme, insbesondere die Verwendung von Verschleißteilen wie Blitzlampen oder in den Kühlsystemen mit flüssigen Kühlmitteln regelmäßige und teils aufwändige Wartung, was ebenfalls nachteilig ist.

Es ist daher eine Aufgabe der vorliegenden Erfindung, ein System für medizinische Behandlungen, insbesondere zur Behandlung der BHP bereitzustellen, welches einen mobilen Einsatz erlaubt.

Es ist eine weitere Aufgabe der vorliegenden Erfindung, ein System für medizinische Behandlungen, insbesondere zur Behandlung der BHP bereitzustellen, welches wartungsarm ist.

Es ist eine nochmals weitere Aufgabe der vorliegenden Erfindung, für eine gegebene medizinische Behandlung, insbesondere der Behandlung von BPH ein System bereitzustellen, das zur bestmöglichen Behandlung unter Minimierung etwaiger Nebeneffekte geeignet und bevorzugt blutungsarm ist, insbesondere durch geeignete Auswahl von Wellenlänge, Leistungsdichte und/oder Pulsdauer bzw. Dauerbetrieb.

Darüber hinaus wird bei heutigen Lasersystemen Lichtleistung entweder kontinuierlich (cw) oder in Form von Laserpulsen abgegeben. Um im cw-Betrieb Gewebe effizient durch Ablation bzw. Vaporisation zu entfernen, ist eine relativ hohe Leistung P_{cw} erforderlich. Diese kann so hoch sein, dass es aufgrund photothermischer Effekte und langer Expositionsdauern zu unerwünscht großen Koagulationssäumen oder gar zur Nekrosebildung kommt. Soll ablativ bzw. vaporisierend Gewebe entfernt werden, wird daher oftmals im gepulsten Betrieb (Pulsdauern im Mikrosekunden- bis Millisekundenbereich) gearbeitet. Hierbei entstehen oft nur geringe Koagulationssäume, so dass nicht blutungsfrei bzw. blutungsarm gearbeitet werden kann, und es besteht die Gefahr von Gewebeperforationen.

Es wäre daher wünschenswert, Gewebe entfernen zu können, ohne dass es zu Blutungen aus umliegenden Gewebearealen bzw. zur Perforation benachbarter Strukturen kommt. Neben der bereits erwähnten urologischen Behandlung der BPH gibt es eine Vielzahl weiterer Anwendungen, die von einer derart vergleichsweise schonenden Behandlung profitieren würden. Diese medizinischen Anwendungen finden sich zum Beispiel auf den Gebieten der allgemeinen Chirurgie, der Gynäkologie, der Urologie, der Tumortherapie. Zwei weitere Beispiele sind:die Tumorresektion und die Thoraxchirurgie.

Bei einer Tumorresektion müssen manche Arten von Tumorgewebe erst koaguliert werden, bevor sie reseziert werden können, um die Gefahr der Metastasenbildung zu verhindern. Bislang wurden vor allem Diodenlaser (z. B. 940nm) bzw. Festkörperlaser (zum Beispiel Nd:YAG, 1064nm) zur gastroskopischen, bronchioskopischen bzw. rektoskopischen Behandlung eingesetzt. Mit zunächst geringer Laserleistung wird Tumorgewebe koaguliert, um anschließend bei hoher Leistung entfernt zu werden. Aufgrund der hohen Eindringtiefe von Laserstrahlung der Wellenlängen 940nm bzw. 1064nm besteht die Gefahr der Perforation umliegender Gewebestrukturen. Unter Umständen wird deshalb bei zu geringer Leistung gearbeitet, um eine solche Perforation zu vermeiden, was eine nicht ausreichende Koagulation zur Folge hat.

In der Thoraxchirurgie wird bei einer heutigen Teilresektion der Lunge Lungengewebe mit einem Festkörperlaser zumeist mit einer Wellenlänge von 1320nm entfernt. Dieser Laser wirkt hauptsächlich ablativ. Alternativ werden seit kurzem Diodenlaser (λ = 980nm) eingesetzt, die ein deutlich besseres Koagulationsverhalten aufweisen. Um bei dieser Wellenlänge jedoch effizient Gewebe abtragen zu können, sind Lichtleistungen von mehr als 80W erforderlich.

Es ist daher eine weitere Aufgabe der Erfindung, ein System für medizinische Behandlungen, insbesondere zur Behandlung der BHP bereitzustellen, welches es ermöglicht, Gewebe zu entfernen, ohne dass es zu Blutungen aus umliegenden Gewebearealen bzw. zur Perforation benachbarter Strukturen kommt.

Diese und andere Aufgaben der vorliegenden Erfindung werden mit einem System gemäß dem Anspruch 1 gelöst. Weitere bevorzugte Ausführungsformen sind in den abhängigen Ansprüchen dargelegt.

Insbesondere erlaubt die vorliegende Erfindung, Systeme mit einem hohen Wirkungsgrad von beispielhaft über 55% bereitzustellen, was es erlaubt, die Kühlung als Luftkühlung zu verwirklichen und auf wartungsintensive, komplexe und schwere Wasserkühlungssysteme zu verzichten. Auf diese Weise können Systeme von vergleichsweise geringem Gewicht, beispielhaft unter 40kg, verwirklicht werden. Der hohe Wirkungsgrad erlaubt es auch, solche Systeme bei einphasigem elektrischem Stromanschluss mit 230V und 10A zu betreiben. Dies hat den Vorteil, dass solche Systeme transportabel und beinahe überall einsetzbar sind, ohne spezielle Stromversorgungen und Kühlsysteme zu erfordern.

Des Weiteren sind solche Systeme, da sie auf verschleißanfällige Teile und Baugruppen wie z. B. Blitzlampen oder Wasserkühlsysteme verzichten, sehr wartungsarm.

Durch einfache, modulare Systemkonzepte können darüber hinaus die erforderlichen Leistungen bei gleichzeitiger Flexibilität aller Behandlungsparameter wie zum Beispiel Leistung, Pulslänge (einige Mikrosekunden bis Sekunden) und Pulspause (einige Mikrosekunden bis Sekunden) erreicht werden.

Solche Systeme besitzen das Potential, BPH-Behandlungen bis zu größten Prostatavolumina mit einem Gewicht von bis zu 200g durchzuführen. Bisher sind keine Kontraindikationen bekannt, so dass beispielsweise auch Patienten behandelt werden können, die Medikamente zur Blutverdünnung einnehmen bzw. die unter Herz-Kreislauf-Schwierigkeiten leiden.

Darüber hinaus erlaubt die vorliegende Erfindung, Systeme für eine kombinierte Bestrahlung mit kontinuierlicher Laseremission und zusätzlichen Laserpulsen bereitzustellen. Dies ist insbesondere Anwendungen vorteilhaft, bei denen Gewebe, insbesondere ein größeres Gewebevolumen blutungsarm entfernt werden sollen. Durch den geringeren Wärmeeintrag aufgrund einer gegenüber herkömmlichen Dauerstrichlasern reduzierten cw-Leistung wird ein kleinerer Bereich koaguliert. In Verbindung mit der höheren Pulsspitzenleistung kann Gewebe präzise vaporisiert werden bei gleichzeitiger Erzeugung eines geringen Koagulationssaumes, der Blutungen verhindert bzw. reduziert.

Diese und andere Vorteile, Merkmale und Eigenschaften der vorliegenden Erfindung werden aus der folgenden Beschreibung ersichtlich, welche allein beispielhaft und nicht beschreibend ist, wobei sich auf die beigefügte Zeichnungen bezogen wird, welche zeigen:
- Fig. 1:: die Absorption von Zellen und Gewebe abhängig von der Wellenlänge;

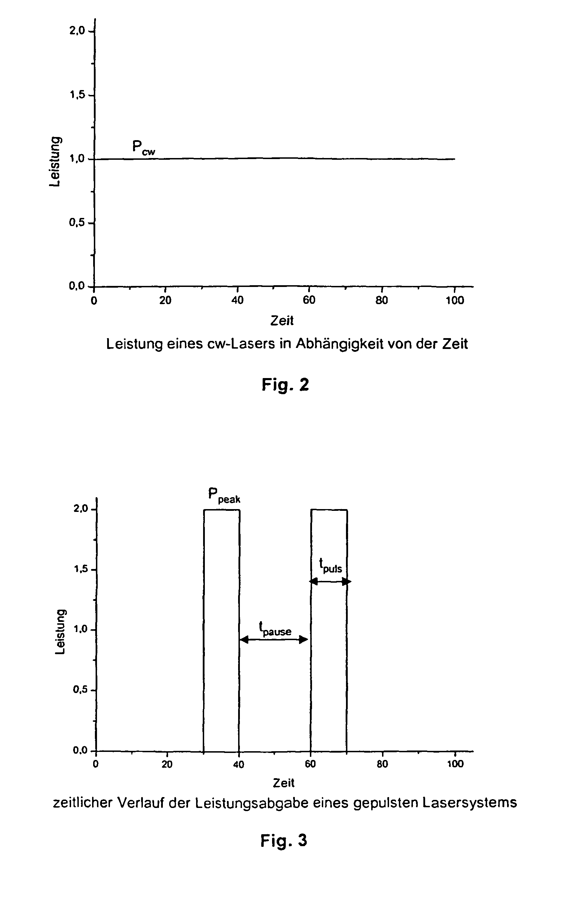

- Fig. 2:: die Leistung eines cw-Lasers in Abhängigkeit von der Zeit;
- Fig. 3:: den zeitlichen Verlauf der Leistungsabgabe eines gepulsten Lasersystems; und
- Fig. 4:: den zeitlichen Verlauf der von einem Laser im "mixed-mode"-Betrieb emittierten Laserleistung.

Gemäß der vorliegenden Erfindung ist ein System für medizinische Behandlungen mit einer Diodenlaservorrichtung zur Erzeugung von Laserstrahlung. sowie einem lichtwellenleiterbasierten Applikationssystem ausgestattet, das endoskopisch in einen Patienten eingeführt werden kann. Die von dem Diodenlaservorrichtung erzeugte Laserstrahlung wird durch das Applikationssystem geleitet und von dem Applikationssystem emittiert, so dass mit der austretenden Laserstrahlung biologisches Gewebe selektiv bearbeitet werden kann, z.B. durch Koagulation, Vaporisation oder Schneiden.

Die in dem System gemäß der Erfindung verwendete Diodenlaservorrichtung ist in der Lage, Strom direkt in zur medizinischen Behandlung verwendbares Laserlicht umzuwandeln. Es entfällt daher die Notwendigkeit, wie bei vorbekannten festkörperlaserbasierten Systemen neben dem eigentlichen Laser selbst noch zusätzlich Blitzlampen oder Laserdioden vorzusehen, die den Laser anregen, so dass ein wesentlich vereinfachter Systemaufbau erzielt werden kann. Zudem haben solche Diodenlaser einen hohen Wirkungsgrad von teilweise über 55%. Dies erlaubt es, auf aufwändige Kühlvorrichtungen, wie zum Beispiel Wasserkühlung zu verzichten.

Daneben sind solche Diodenlaser mit Wellenlängen im Spektralbereich zwischen 800 bis 2000nm verfügbar, was es erlaubt, durch geeignete Wahl der Wellenlänge und/oder Leistungsdichte Systeme zur medizinischen Behandlung bereitzustellen, die für eine gegebene Behandlung sozusagen "maßgeschneidert" sind, bei gleichzeitig hohen Leistungen und guten Lebensdauern von über 3000 Betriebsstunden. Als Diodenlaser können einzelne Laserdioden oder Laserdiodenbarren verwendet werden.

Vorzugsweise weist das Applikationssystem ein urologisches Instrument auf, zum Beispiel ein Zysto-Urethroskop, in welchem eine geeignete Glasfaser geführt wird, die an den lichtemittierenden Ausgang des Diodenlasersystems angeschlossen wird. Laserlicht wird in den Faserkern der Glasfaser eingekoppelt, durch den Faserkern bis zum distalen Ende der Glasfaser geleitet und dort abgestrahlt.

Bevorzugt ist die Glasfaser mit zwei Vorrichtungen versehen, die die Möglichkeit zur Vorwärtsbewegung bzw. Rückwärtsbewegung im urologischen Instrument begrenzen. Hierdurch wird ein zu weites Herausschieben der Faser aus dem urologischen Instrument verhindert, wodurch die Gefahren eines Faserbruches bzw. einer Gewebeperforation durch mechanische Einwirkung der Glasfaser deutlich reduziert werden. Die Einschränkung der Bewegungsfreiheit in rückwärtiger Richtung dient unter anderem dem Schutz endoskopischer Optiken, die in das urologische Instrument integriert sind bzw. dem Schutz des Instruments selber. Bevorzugt weist eine solche Glasfaser die folgenden Werte auf:

### Faserparameter

- Kerndurchmesser Ø_{core} ≥ 400µm
- Numerische Apertur NA = 0,22
- Faserkern aus Quarzglas
- Jacket aus biokompatiblen Material, z. B. Tefzel

Als Alternative dazu kann in einem sogenannten "side-fiber"-Applikationssystem eine Quarzglasfaser verwendet werden, welche die Laserstrahlung nicht über die distale Stirnfläche, sondern über einen kleinen Bereich der Manteloberfläche abgibt. Dabei wird diese "side fiber" an den lichtemittierenden Ausgang des Diodenlasers angeschlossen. Laserlicht wird in den Faserkern eingekoppelt, durch den Faserkern bis zum distalen Ende der Glasfaser geleitet und dort durch dessen Manteloberfläche seitwärts gerichtet abgestrahlt. Die Glasfaser wird in einem urologischen Instrument, z. B. einem Zysto-Urethroskop geführt. Eine entsprechende Glasfaser ist zum Beispiel in dem Dokument WO2007/058891 offenbart, welches lediglich eine Diodenlaser vorrichtung zeigt und auf das für weitere Details des Aufbaus einer solchen "side fiber" verwiesen wird. Bevorzugt weist auch diese Glasfaser die bereits oben genannten Faserparameter auf.

In einer ersten besonderen Ausführungsform der vorliegenden Erfindung wird ein Diodenlasersystem bei einer Wellenlänge von 980nm ± 30nm mit einer mittleren Leistung von mehr als 100W eingesetzt. Die Laserstrahlung wird in eine Glasfaser mit 400µm Kerndurchmesser oder mehr eingekoppelt.

Ein Vorteil der Verwendung einer Wellenlänge von 980nm ist es, dass damit durch die gleichermaßen gute Absorption in Wasser und in Blut eine Ablation erzielt wird, die mit dem Ablationsverhalten des KTP-Lasers vergleichbar ist. Das Gewebe wird oberflächlich beseitigt und darüber hinaus in der Tiefe von einigen Millimetern koaguliert, so dass die dort befindlichen arteriellen Gefäße oder Venen verschlossen werden. So ist das Auftreten von Blutungen nahezu ausgeschlossen. Dies wird als großer Vorteil angesehen, da die Notwendigkeit einer Bluttransfusion, wie sie bei der TURP nötig sein kann, auch heutzutage noch als kritisch angesehen werden muss. Eine Verringerung des intra- und postoperativen Blutverlustes stellt daher nach wie vor einen der wichtigsten Punkte in der chirurgischen Behandlung der BPH dar.

Durch die große Vielzahl zur Verfügung stehender medizinischer Fasern und Lichtwellenleiter-basierter Applikationssysteme sind auch Anwendungen im oberen Harntrakt möglich (Polamputation).

Die Wirksamkeit dieser ersten besonderen Ausführungsform wurde in ersten Versuchen bestätigt. Dabei hat sich auch gezeigt, dass die Leistung bis auf etwa 60W reduziert werden kann; allerdings geht dies zu Lasten der Bearbeitungsgeschwindigkeit und unter Umständen des Therapieergebnisses. Nachstehend ist der in den Versuchen verwendete Behandlungsparametersatz wiedergegeben:

### Behandlungsparametersatz

- Pulsdauer (Zeit, in der der Laser Leistung abgibt):
   10ms oder 100ms oder 200ms oder 100ms
- Pulspause (Zeitraum zwischen zwei Laserpulsen):
   10ms
- mittlere Laserleistung:
   100 - 250W
- Faserparameter
   Siehe oben.

In einer zweiten besonderen Ausführungsform wird die erste besondere Ausführungsform dahingehend abgewandelt, dass nun ein Diodenlaser mit einer Emissionswellenlänge von 940nm ± 30nm eingesetzt wird, dessen Laserstrahlung in eine Glasfaser mit 400µm Kerndurchmesser oder mehr eingekoppelt wird.

### Behandlungsparametersatz

- Pulsdauer (Zeit, in der der Laser Leistung abgibt):
   10ms oder 100ms oder 200ms oder 1000ms
- Pulspause (Zeitraum zwischen zwei Laserpulsen):
   10ms
- mittlere Laserleistung:
   100 - 250W
- Faserparameter
   Siehe oben

In einer dritten besonderen Ausführungsform wird die erste besondere Ausführungsform dahingehend abgewandelt, dass ein Diodenlasersystem mit einer Emissionswellenlänge von 810nm ± 30nm eingesetzt wird, dessen Laserstrahlung in eine Glasfaser mit 400µm Kerndurchmesser oder mehr eingekoppelt wird.

### Behandlungsparametersatz

- Pulsdauer (Zeit, in der der Laser Leistung abgibt):
   10ms oder 100ms oder 200ms oder 1000ms
- Pulspause (Zeitraum zwischen zwei Laserpulsen):
   10ms
- mittlere Laserleistung:
   ≥ 100W
- Faserparameter
   Siehe oben

In einer vierten besonderen Ausführungsform wird die erste besondere Ausführungsform dahingehend abgewandelt, dass nun ein Diodenlaser mit einer Emissionswellenlänge von 1470nm verwendet. Die Wahl dieser Emissionswellenlänge hat sich in Versuchen als besonders interessant herausgestellt, da bei dieser Wellenlänge die Eindringtiefe in Wasser durch den im Vergleich zu der Wellenlänge λ = 980nm 30fach höheren Absorptionskoeffizient stark reduziert ist.

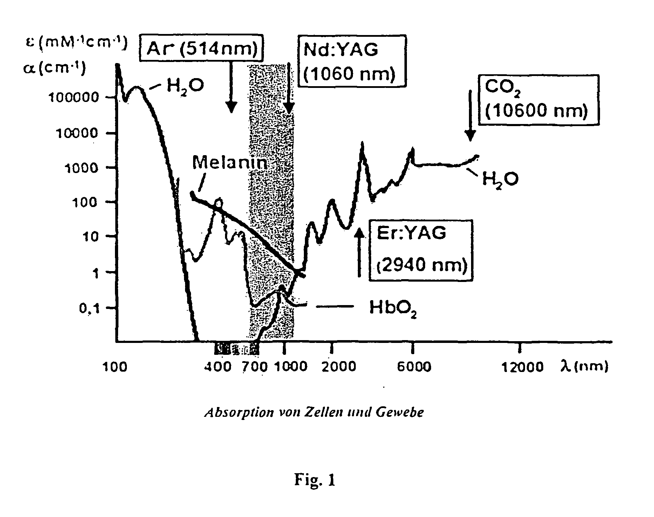

Dieses von der Wellenlänge abhängige Absorptionsverhalten und damit auch die wellenlängenabhängige Eindringtiefe der Hauptgewebebestandteile Wasser H₂O, Hämoglobin HbO₂ und Melanin, ist in der Figur 1 dargestellt, wobei im Falle der Urologie die beiden erstgenannten Substanzen hauptsächlich relevant sind. In der Figur 1 stellt die vertikale Skala mit den Werten α(cm⁻¹) den Absorptionskoeffizienten für Wasser bzw. Melanin dar. Die Skala ε(mM⁻¹cm⁻¹) stellt den konzentrationsabhängigen Absorptionskoeffizienten für Hämoglobin (HbO₂) in der Einheit pro millimol pro cm dar. Die Eindringtiefe ist der Kehrwert des Absorptionskoeffizienten. Eine geringere Eindringtiefe bedeutet, dass die Laserstrahlung bereits früher absorbiert wird und nicht so tief in das Gewebe, welches bekanntlich zu einem Großteil aus Wasser besteht, eindringt. Dementsprechend wird auch nur ein kleineres Gewebevolumen bearbeitet. Eine verminderte Eindringtiefe erlaubt somit, dass präziser gearbeitet werden kann und die Zone der Kollateralschäden ist geringer.

Es wurden erste Versuche an Hundeprostaten vorgenommen, um die Wirkungsweise von 1470nm in Diodenlasertechnologie zu prüfen. Hier erwiesen sich mittlere Leistungen von ca. 40W bereits als ausreichend für eine selektive Gewebevaporisation.

### Behandlungsparametersatz

- Pulsdauer (Zeit, in der der Laser Leistung abgibt):
   100ms
- Pulspause (Zeitraum zwischen zwei Laserpulsen):
   10ms
- mittlere Laserleistung:
   40W
- Faserparameter
   Siehe oben

In einer weiteren besonderen Ausführungsform wird ein Diodenlaser mit einer Wellenlänge von λ = 1,95µm = 1950nm verwendet. Hier sind derzeit passiv gekühlte Laserdiodenbarren mit einer Leistung im 10W-Bereich verfügbar. Modular werden hier Systeme für urologische Anwendungen aufgebaut, die eine mittlere Leistung von ca. 50W erreichen.

Laserstrahlung dieser Wellenlänge wirkt weniger koagulierend, dafür aber präzise schneidend. Durch die hohe Absorption von Wasser in diesem Spektralbereich wird jedoch Wasser durch die absorbierte Laserstrahlung so stark erhitzt, dass es zu Dampfblasen kommen kann, die ihrerseits dann zu einer Gewebekoagulation führen können.

Derzeit werden lampen- bzw. diodengepumpte Thulium- und Holmium-Festkörperlaser eingesetzt. Urologische Thulium-Festkörperlaser emittieren bei einer Wellenlänge von 2040nm. Der Absorptionskoeffizient ist hierbei ca. 2,5mal höher als bei einer Wellenlänge λ=2140nm, wie sie von Holmium-Festkörperlasern erzeugt wird.

Im Vergleich dazu profitieren bei 1950nm emittierende Diodenlaser nicht nur von der gegenüber der Thulium-Laserwellenlänge 1,5fach besseren Absorption, sondern auch davon, dass sich bei steigenden Gewebetemperaturen das Wasserabsorptionsmaximum zu kürzeren Wellenlänge verschiebt. Es wird daher erwartet, dass im Vergleich zur Emission von Thulium- und Holmium-Lasern eine noch bessere Absorption der Diodenlaserstrahlung erreicht werden kann.

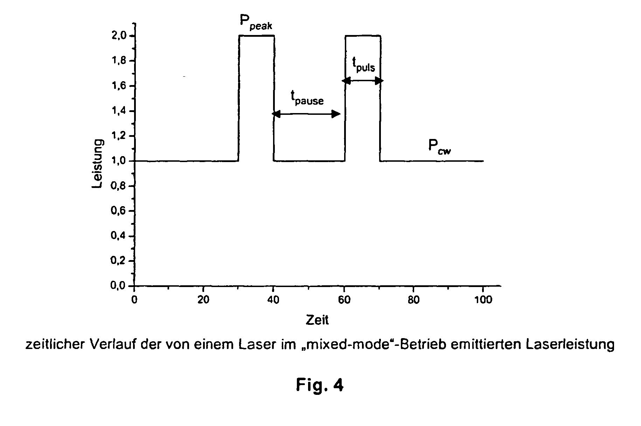

Die in dem System gemäß der Erfindung verwendete Diodenlaservorrichtung kann darüber hinaus in einem hierin als "mixed mode" bzw. gemischten Modus bezeichneten Betriebsmodus arbeiten. Dabei wird Laserleistung als eine Kombination von kontinuierlicher Leistungsabgabe in Verbindung mit Laserpulsen geeigneter Pulsdauer emittiert, so dass sich eine resultierende emittierte Laserleistung ergibt, wie sie allein schematisch im zeitlichen Verlauf in der Fig. 4 gezeigt ist.

Bei diesem "mixed mode" Betrieb sind bevorzugt sowohl die cw-Leistung P_{cw} als auch die Pulsspitzenleistung Pₚₑₐₖ unabhängig voneinander einstellbar. Das Verhältnis P_{cw}/Pₚₑₐₖ ist bevorzugt ebenfalls variabel wählbar. Besonders bevorzugt sind die Pulsdauer tₚᵤₗₛ, die Pulspause tₚₐᵤₛₑ, die Zeit zwischen dem Ende eines Pulses und dem Beginn des nächsten Pulses definiert ist, sowie die Anzahl der Laserpulse wählbar.

Die bevorzugte Pulsdauer solcher Laserpulse, die ablativ/vaporisierend wirken, ist kürzer als die Zeitkonstante der Wärmeleitung des Zielgewebes. Typische Pulsdauern liegen im Bereich 0,01 - 100ms.

Bevorzugte Wellenlängen eines solchen Lasersystems liegen im Spektralbereich 800 bis 1000nm. Besonders bevorzugt sind die Wellenlängen 810, 940 und 980nm.

Ein weiterer bevorzugter Wellenlängenbereich liegt zwischen 1400nm und 1500nm. Eine besonders bevorzugte Wellenlänge ist 1470nm.

Der "mixed-mode"-Betrieb erlaubt durch Kombination von kontinuierlicher Leistungsabgabe in Verbindung mit Laserpulsen geeigneter Pulsdauer und Pulsspitzenleistungen eine effiziente Gewebeentfernung bei gleichzeitiger Ausbildung eines Koagulationssaumes, der Blutungen verhindert bzw. weitestgehend einschränkt. Durch Wahl der Laserparameter (P_{cw}, Pₚₑₐₖ, tₚᵤₗₛ, tₚₐᵤₛₑ) lassen sich Ablations- und Koagulationsverhalten beeinflussen.

Die Laserpulse mit der höheren Spitzenleistung (Pₚₑₐₖ > P_{cw}) führen zur Ablation/Vaporisation des Gewebes. Aufgrund der Pulsdauer bzw. der Pulspausen wird unnötige thermische Belastung des Zielgewebes und der umliegenden Gewebeareale vermieden. Eine solche thermische Belastung kann unter Umständen zu unerwünschter Nekrosebildung führen. Die gleichzeitige kontinuierliche Leistungsabgabe (P_{cw}) führt jedoch zur Bildung eines koagulierten Bereiches, so dass Gewebe durch die Laserpulse blutungsfrei bzw. blutungsarm entfernt werden kann.

Neben der bereits erwähnten urologischen Behandlung der BPH gibt es eine Vielzahl weiterer Anwendungen, die von der Erfindung des "mixed-mode"-Betriebes profitieren. Hierbei soll Gewebe entfernt werden, ohne dass es zu Blutungen aus umliegenden Gewebearealen bzw. zur Perforation benachbarter Strukturen kommt. Diese medizinischen Anwendungen finden sich zum Beispiel auf den Gebieten der allgemeinen Chirurgie, der Gynäkologie, der Urologie, der Tumortherapie. Zwei weitere Beispiele sind die Thoraxchirurgie und die Tumorresektion.

Eine kombinierte Bestrahlung mit kontinuierlicher Laseremission und zusätzlichen Laserpulsen ist für die Anwendung in der Thoraxchirurgie und hier insbesondere der Teilresektion der Lunge vorteilhaft, da blutungsarm größere Gewebevolumina entfernt werden könnten. Durch den geringeren Wärmeeintrag (aufgrund der gegenüber herkömmlichen Dauerstrichlasern reduzierten cw-Leistung) wird ein kleinerer Bereich koaguliert. In Verbindung mit der höheren Pulsspitzenleistung kann Gewebe präzise vaporisiert werden bei gleichzeitiger Erzeugung eines geringen Koagulationssaumes, der Blutungen verhindert bzw. reduziert.

Bei der Tumorresektion erlaubt ein Lasergerät mit der Möglichkeit eines "mixed-mode"-Betriebs einen neuen Behandlungsansatz: Zunächst erfolgt die Koagulation des Tumorgewebes im non-Kontakt-Verfahren beispielsweise bei einer Wellenlänge von 980nm mit Leistungen von 50 - 80W. Das so thermisch vorbehandelte Gewebe kann dann anschließend durch Laservaporisation effizient und sicher entfernt werden.

Während im Vorhergehenden Systeme mit einer Diodenlaservorrichtung zur Erzeugung von Laserstrahlung einer Wellenlänge beschrieben wurden, ist es in einer weiteren, besonderen Ausführungsform auch möglich, ein System mit einer zusätzlichen, zweiten Diodenlaservorrichtung bereitzustellen, so dass das System Laserlicht mit zwei unterschiedlichen Wellenlängen (λ₁, λ₂) erzeugt, welche verschiedene Eindringtiefen in biologischem Gewebe aufweisen.

Das Licht beider Strahlquellen wird durch optische Mittel, wie zum Beispiel einen dichroitischen Spiegel, kombiniert, zum Beispiel kollinear überlagert, so dass die Laserstrahlung beider Wellenlängen in ein gemeinsames Applikationssystem eingekoppelt werden kann. Durch das Applikationssystem wird Licht beider Wellenlängen zu demselben Behandlungsort geleitet, so dass ein und dasselbe Zielgewebe mit einer der beiden Wellenlängen oder simultan mit beiden Wellenlängen bestrahlt und damit therapiert werden kann.

Dabei kann zum Beispiel Licht der ersten Wellenlänge λ₁ zur Koagulation des Gewebes eingesetzt werden, während Licht der Wellenlänge λ₂ zur Ablation/Vaporisation des Gewebes dient und somit zum präzisen Schneiden eingesetzt werden kann.

Durch Variation der zugehörigen (cw-) Leistungen P₁ und P₂ sowie des Verhältnisses P₁/P₂ können Koagulationssaum und Schnitttiefe und ihr Verhältnis zueinander beeinflusst und der jeweiligen medizinischen Anwendung angepasst werden. P₁ und P₂ können unabhängig voneinander gewählt werden.

Dabei können die Leistungen P₁ entsprechend der Wellenlänge λ₁ und P₂ entsprechend der Wellenlänge λ₂ und deren Verlauf über die Zeit auch so gewählt werden, dass eine Laserquelle kontinuierlich Leistung (cw-Betrieb) abgibt, während die zweite Strahlquelle mit gepulster Leistungsabgabe betrieben wird. Für einen solchen "mixed-mode"-Betrieb mit mehrere Wellenlängen ist die Kombination von 980nm- und 1470nm-Laserstrahlung eine bevorzugte Wahl.

Es können die Leistungen P₁ und P₂ auch so gewählt werden, dass beide Laserquellen kontinuierlich Leistung (cw-Betrieb) abgeben, oder die Leistungen P₁ und P₂ können so gewählt werden, dass beide Strahlquellen im Pulsmodus betrieben wird. Alternativ ist es auch möglich, dass eine der Laserquellen im "mixed mode" Betrieb arbeitet, während die andere Laserquelle nur kontinuierlich Leitung (cw-Betrieb) abgibt oder nur im Pulsmodus betrieben wird.

Es ist dabei bevorzugt, dass die Wellenlänge λ₁ aus dem Spektralbereich 800 - 1100nm gewählt, während λ₂ aus dem Wellenlängenbereich 1400 bis 1500nm oder 1900 bis 2000nm gewählt wird. Besonders bevorzugt ist die Wellenlängenkombination λ₁ = 980nm und λ₂ = 1470nm.

Während heute für urologische Anwendungen Leistungen von mehr als 100W bei einer Wellenlänge von 980nm eingesetzt werden, lassen sich durch den Einsatz eines Systems mit zwei Diodenlaservorrichtungen, welche Licht bei zwei Wellenlängen emittieren, die für Ablation/Vaporisation und Koagulation notwendigen Leistungen genauer dosieren und die unterschiedlichen Wechselwirkungseigenschaften von λ₁ und λ₂ mit Gewebe vorteilhaft ausnutzen. Eine Gesamtleistung P₁ + P₂ < 100W wird erwartet.

Die Kombination zweier Laserwellenlängen bei geeigneter Wahl der Leistungen P₁ und P₂ sowie des Verhältnisses beider Leistungen erlaubt eine medizinisch vorteilhafte Wirkung von Ablation (effiziente aber präzise Entfernung auch größerer Gewebevolumina) und Koagulation (blutungsame bzw. blutungsfreie Operation). Dies ist für den Patienten insofern vorteilhaft, als die Behandlung effizienter und somit schneller bei gleichzeitig größerer Sicherheit durchgeführt werden kann.

Koagulation durch die erste der beiden Laserwellenlängen reduziert das Risiko einer Blasenwandperforation, wie sie bei primär ablativ wirkenden Lasern (zum Beispiel KTP-Laser) auftreten kann.

Die Nutzung einer ablativ wirkenden Wellenlänge verringert ggf. die Nebenwirkungen, die beim Einsatz rein sekundär ablativ wirkender Lasersysteme (zum Beispiel Nd:YAG-Laser) beobachtet werden, insbes. die Gefahr der Nekrosenbildung, die den Heilungsprozess verlängert.

Der Anwender (Arzt) kann beide Wellenlängen einzeln oder gleichzeitig einsetzen und so schneiden bzw. koagulieren, ohne Lasergerät und/oder Applikationssystem wechseln zu müssen. Letzteres ist insbesondere bei Anwendungen im Körper des Patienten vorteilhaft.

## Patentansprüche

1. System für medizinische Behandlungen, aufweisend eine erste Diodenlaservorrichtung zur Erzeugung von Laserstrahlung einer ersten Wellenlänge λ₁ und eine zweite Diodenlaservomichtung zur Erzeugung von Laserstrahlung einer zweiten Wellenlänge λ₂ sowie ein lichtwellenleiterbasiertes Applikationssystem, welches endoskopisch in einen Patienten einführbar ist, wobei das System so eingerichtet ist, dass von der ersten und der zweiten Diodenlaservorrichtung erzeugte Laserstrahlung durch das Applikationssystem leitbar und von dem Applikationssystem emittier bar ist, so dass mit der austretenden Laserstrahlung biologisches Gewebe selektiv bearbeit bar ist, wobei die Wellenlänge λ₁ aus dem Spektralbereich 800 bis 1100nm gewählt ist und die Wellenlänge λ₂ aus dem Spektralbereich 1400 bis 1500nm oder 1900 bis 2000nm gewählt ist.

2. System nach Anspruch 1, wobei die selektive Bearbeitung von biologischem Gewebe durch Koagulation, Vaporisation oder Schneiden erfolgt.

3. System nach einem der Ansprüche 1 bis 2, welches eingerichtet ist, die Laserstrahlung über das eingesetzte Applikationssystem auf das Zielgewebe mit einer Leistungsdichte von mehr als 5kW / cm² für Wellenlängen im Bereich von 800 bis 1100nm bzw. mit einer Leistungsdichte von mehr als 3kW / cm² für Wellenlängen im Bereich von 1400 bis 1500nm übertragen zu können.

4. System nach einem der Ansprüche 1 bis 2, wobei die erste Diodenlaservorrichtung eine mittlere Laserleistung von mehr als 100W bei einer Wellenlänge im Bereich von 800 bis 1100nm aufweist, wobei diese Leistung im Dauerstrichbetrieb oder durch mehrere Pulse mit einer Pulsdauer im Bereich einiger Mikrosekunden bis einiger Sekunden abgegeben werden kann.

5. System nach einem der Ansprüche 1 bis 3, wobei die zweite Diodenlaservorrichtung eine mittlere Laserleistung von mehr als 50W bei einer Wellenlänge im Bereich von 1900 bis 2000nm aufweist, wobei diese Leistung im Dauerstrichbetrieb oder durch mehrere Pulse mit einer Pulsdauer im Bereich einiger Mikrosekunden bis einiger Sekunden abgegeben werden kann.

6. System nach einem der Ansprüche 1 bis 3, wobei die zweite Diodenlaservorrichtung eine mittlere Laserleistung von mehr als 40W bei einer Wellenlänge im Bereich von 1400 bis 1500nm aufweist, wobei diese Leistung im Dauerstrichbetrieb oder durch mehrere Pulsen mit einer Pulsdauer im Bereich einiger Mikrosekunden bis einiger Sekunden abgegeben werden kann.

7. System nach einem der vorhergehenden Ansprüche, wobei das Applikationssystem eine Quarzglasfaser aufweist, derart eingerichtet, die Laserstrahlung nicht über die distale Stirnfläche, sondern über einen kleinen Bereich der Manteloberfläche abzugeben.

8. System nach einem der vorhergehenden Ansprüche, wobei das Applikationssystem eine Quarzglasfaser aufweist, derart eingerichtet, das zwei Vorrichtungen die Möglichkeit zur Vorwärts- und Rückwärtsbewegung in einem ärztlichen Instrument begrenzen.

9. System nach einem der vorhergehenden Ansprüche, wobei das System so eingerichtet ist, dass die erste und/oder die zweite Diodenlaservorrichtung im gemischten Modus arbeitet bzw. arbeiten unter Emission von Laserstrahlung in Form einer Kombination von kontinuierlicher Leistungsabgabe in Verbindung mit Laserpulsen.

10. System nach Anspruch 9, wobei die kontinuierliche Leistung P_{cw} als auch die Pulsspitzenleistung Pₚₑₐₖ unabhängig voneinander einstellbar sind, insbesondere das Verhältnis P_{cw}/Pₚₑₐₖ variabel wählbar ist.

11. System nach Anspruch 9 oder 10, wobei die Pulsdauer tₚᵤₗₛ, die Pulspause tₚₐᵤₛₑ sowie die Anzahl der Laserpulse variabel wählbar sind.

12. System nach einem der Ansprüche 9 bis 11, wobei die erste Wellenlänge bevorzugt eine der Wellenlängen von 810, 940 und 980nm ist.

13. System nach einem der Ansprüche 9 bis 11, wobei die Laserstrahlung eine Wellenlänge im Spektralbereich von 1400 bis 1500nm aufweist, wobei die Wellenlänge bevorzugt 1470nm ist

14. System nach einem der vorhergehenden Ansprüche zur Behandlung der BPH, zur Anwendung bei einer Tumorresektion oder zur Anwendung in der Thoraxchirurgie.

## Claims

1. A system for medicinal treatment, having a first diode laser device for producing a laser beam of a first wavelength λ₁ and a second diode laser device for producing a laser beam of a second wavelength λ₂ and having an application system based on optical wave guides that can be endoscopically introduced into a patient, wherein the system is designed in such a manner that the laser beam produced by the first and the second diode laser device can be guided through the application system and can be emitted by said application system so that biological tissue can be treated in a selective manner with the emerging laser beam, wherein the wavelength λ₁ is selected from the spectral range 800 to 1100 nm and the wavelength λ₂ is selected from the spectral range 1400 to 1500 nm or 1900 to 2000 nm.

2. The system according to claim 1, wherein biological tissue is selectively treated through coagulation, vaporisation or cutting.

3. The system according to either of claims 1 and 2, which is designed to be able to transmit the laser beam via the inserted application system to the target tissue with a power density of more than 5 kW/cm² for wavelengths in the range from 800 to 1100 nm and with a power density of more than 3 kW/cm² for wavelengths in the range from 1400 to 1500 nm.

4. The system according to either of claims 1 and 2, wherein the first diode laser device has an average laser power of more than 100 W at a wavelength in the range from 800 to 1100 nm, wherein this power can be emitted in continuous beam operation or through multiple pulses with a pulse duration in the range from a few microseconds to a few seconds.

5. The system according to any of claims 1 to 3, wherein the second diode laser device has an average laser power of more than 50 W at a wavelength in the range from 1900 to 2000 nm, wherein this power can be emitted in continuous beam operation or through multiple pulses with a pulse duration in the range from a few microseconds to a few seconds.

6. The system according to any of claims 1 to 3, wherein the second diode laser device has an average laser power of more than 40 W at a wavelength in the range from 1400 to 1500 nm, wherein this power can be emitted in continuous beam operation or through multiple pulses with a pulse duration in the range from a few microseconds to a few seconds.

7. The system according to any of the preceding claims, wherein the application system has a quartz glass fibre designed to emit the laser beam not via the distal end face, but instead via a small area of the sleeve surface.

8. The system according to any of the preceding claims, wherein the application system has a quartz glass fibre designed such that two devices limit the possibility of movement forwards and backwards in a medical instrument.

9. The system according to any of the preceding claims, wherein the system is designed in such a manner that the first and/or the second diode laser device operates or operate in mixed mode emitting a laser beam in the form of a combination of continuous power emission and laser pulses.

10. The system according to claim 9, wherein the continuous power P_{cw} and the pulse peak power Pₚₑₐₖ can be set independently of one another, it being possible to variably select the P_{cw}/Pₚₑₐₖ ratio in particular.

11. The system according to claim 9 or 10, wherein the pulse duration tₚᵤₗₛₑ, the pulse pause tₚₐᵤₛₑ and the number of laser pulses can be variably selected.

12. The system according to any of claims 9 to 11, wherein the first wavelength is preferably one of the wavelengths 810, 940 or 980 nm.

13. The system according to any of claims 9 to 11, wherein the laser beam has a wavelength in the spectral range from 1400 to 1500 nm, wherein the wavelength is preferably 1470 nm.

14. The system according to any of the preceding claims for treating BPH, for use in the event of tumour resection or for use in thoracic surgery.

## Revendications

1. Système pour traitements médicaux, comprenant un premier dispositif laser à diode pour générer un faisceau laser d'une première longueur d'onde λ₁ et un deuxième dispositif laser à diode pour générer un faisceau laser d'une deuxième longueur d'onde λ₂ ainsi qu'un système d'application à fibre optique, lequel peut être introduit dans un patient par endoscopie, le système étant conçu de manière que le faisceau laser généré par le premier et le deuxième dispositif laser à diode puisse être guidé à travers le système d'application et être émis par le système d'application, de façon que le faisceau laser sortant permette de traiter sélectivement un tissu biologique, la longueur d'onde λ₁ étant choisie dans la plage spectrale comprise entre 800 et 1100 nm et la longueur d'onde λ₂ étant choisie dans la plage spectrale comprise entre 1 400 nm et 1 500 nm ou 1 900 nm et 2 000 nm.

2. Système selon la revendication 1, dans lequel le traitement sélectif d'un tissu biologique est réalisé par coagulation, vaporisation ou coupe.

3. Système selon une des revendications 1 à 2, lequel est conçu pour pouvoir transmettre le faisceau laser au tissu cible, par l'intermédiaire du système d'application utilisé, avec une densité de puissance supérieure à 5 kW / cm² pour les longueurs d'onde comprises entre 800 et 1100 nm, respectivement avec une densité de puissance supérieure à 3 kW / cm² pour les longueurs d'onde comprises entre 1400 et 1 500 nm.

4. Système selon une des revendications 1 à 2, dans lequel le premier dispositif laser à diode possède une puissance laser moyenne supérieure à 100 W à une longueur d'onde comprise entre 800 et 1100 nm, cette puissance pouvant être délivrée en ondes continues ou par plusieurs impulsions d'une durée d'impulsion comprise entre quelques microsecondes et quelques secondes.

5. Système selon une des revendications 1 à 3, dans lequel le deuxième dispositif laser à diode possède une puissance laser moyenne supérieure à 50 W à une longueur d'onde comprise entre 1 900 et 2 000 nm, cette puissance pouvant être délivrée en ondes continues ou par plusieurs impulsions d'une durée d'impulsion comprise entre quelques microsecondes et quelques secondes.

6. Système selon une des revendications 1 à 3, dans lequel le deuxième dispositif laser à diode possède une puissance laser moyenne supérieure à 40 W à une longueur d'onde comprise entre 1 400 et 1 500 nm, cette puissance pouvant être délivrée en ondes continues ou par plusieurs impulsions d'une durée d'impulsion comprise entre quelques microsecondes et quelques secondes.

7. Système selon une des revendications précédentes, dans lequel le système d'application présente une fibre de verre de quartz conçue de façon à délivrer le faisceau laser non par la surface frontale distale, mais par une petite zone de la surface de la gaine.

8. Système selon une des revendications précédentes, dans lequel le système d'application présente une fibre de verre de quartz conçue de façon que deux dispositifs limitent la possibilité de mouvement en avant et en arrière dans un instrument médical.

9. Système selon une des revendications précédentes, lequel système est conçu de façon que le premier et/ou le deuxième dispositif laser à diode travaillent en mode mixte en émettant un faisceau laser sous la forme d'une combinaison d'une émission continue de puissance associée à des impulsions laser.

10. Système selon la revendication 9, dans lequel la puissance continue P_{cw} ainsi que la puissance de crête des impulsions Pₚₑₐₖ sont réglables indépendamment l'une de l'autre, en particulier le rapport P_{cw}/Pₚₑₐₖ est variable à volonté.

11. Système selon la revendication 9 ou 10, dans lequel la durée d'impulsion tₚᵤₗₛ, la pause d'impulsion tₚₐᵤₛₑ ainsi que le nombre d'impulsions laser sont variables à volonté.

12. Système selon une des revendications 9 à 11, dans lequel la première longueur d'onde est de préférence une des longueurs d'onde parmi 810, 940 et 980 nm.

13. Système selon une des revendications 9 à 11, dans lequel le faisceau laser possède une longueur d'onde dans la plage spectrale comprise entre 1 400 et 1 500 nm, la longueur d'onde étant de préférence de 1 470 nm.

14. Système selon une des revendications précédentes, destiné au traitement de l'HBP, à une utilisation pour une résection tumorale ou à une utilisation en chirurgie thoracique.
